# EUROPEAN PATENT SPECIFICATION

(11) **EP 2 616 456 B1**
(45) Date of publication and mention of the grant of the patent: **26.11.2014**
(21) Application number: 11755361.0
(22) Date of filing: 16.09.2011
(51) Int. Cl.: C07D 333/48, C07D 333/68, C07D 333/78, A61K 31/381, A61P 35/00, A61P 31/12

(54) **NUCLEAR EXPORT INHIBITORS**
KERNEXPORT-INHIBITOREN
INHIBITEURS D'EXPORTATION DE NOYAU

(30) Priority: 16.09.2010 EP 10177212
(43) Date of publication of application: 24.07.2013
(73) Proprietor: Universitätsmedizin der Johannes Gutenberg-Universität Mainz, 55131 Mainz (DE)
(72) Inventor: STAUBER, Roland, 55131 Mainz (DE); FETZ, Verena, 55131 Mainz (DE); KNAUER, Shirley K., 55131 Mainz (DE); MANN, Wolf, 55131 Mainz (DE)
(74) Representative: Reitstötter Kinzebach
(86) International application number: PCT/EP2011/066129
(87) International publication number: WO 2012/035147

(56) References cited:
- WO-A1-2008/102881
- WO-A2-2007/033214

## Description

The invention relates to compounds for use in the treatment or prophylaxis of cancer or viral diseases.

Trafficking between nuclear and cytoplasmic compartments is indispensable for proper cell function, affecting a range of processes in human health and disease (Faustino et al., 2007; Kau et al., 2004; Knauer et al., 2005, and references therein). Transport of macromolecules is strictly controlled by specific signals and transport factors, including proteins of the nuclear pore complex (nucleoporins), the RanGTPase, transport receptors, and specialized molecules that promote the transport of specific protein/RNA complexes (D'Angelo et al., 2009; Fahrenkrog and Aebi, 2003; Zaidi et al., 2007). The best characterized nuclear export signals (NESs) consist of a short leucine-rich stretch of amino acids and interact with the export receptor Exportin 1, also termed CRM1 (Faustino et al., 2007; Knauer et al., 2005, and references therein). The CRM1-cargo complex is then actively transported across the nuclear pore complex to the cytoplasm where the cargo is released after RanGAP-catalyzed GTP hydrolysis (Faustino et al., 2007; Fetz et al., 2009; Kau et al., 2004; Knauer et al., 2005, and references therein). Leucine-rich NESs have been identified in an increasing number of disease relevant cellular and viral proteins implicated in control of transcription, apoptosis, cell cycle, and RNA transport (Cullen, 2003; Faustino et al., 2007; Fetz et al., 2009; Hurt and Silver, 2008; Kau et al., 2004; Knauer et al., 2005; Kratzer et al., 2000; Pavlakis and Stauber, 1998). Since regulated subcellular localization provides an attractive way to control the activity and stability of regulatory proteins and RNAs, interfering with nucleo-cytoplasmic transport in general as a novel therapeutic principle has recently attracted major interest by academia and industry (reviewed in Fetz et al., 2009; Kau et al., 2004).

Many important tumor suppressors and transcription factors protect cells by regulating cell growth and apoptosis. Their deregulated dynamic intracellular localization can serve as an inactivation mechanism resulting in uncontrolled growth and the onset of disease (Faustino et al., 2007; Kau et al., 2004; Knauer et al., 2005, and references therein). One strategy to prevent cytoplasmic localization of important regulators is to inhibit the proteins responsible for their nuclear export. The exportin CRM1 is absolutely required for the nuclear export of a wide variety of cancer-related "shuttle proteins" including p53, B23, STATs, c-Abl, Survivin and FOXOs (Brown et al., 2009; Faustino et al., 2007; Kau et al., 2004; Knauer et al., 2005; Kramer et al., 2009; Stauber et al., 2007, and references therein). Notably, the CRM1-NES interaction of several proteins also plays an important functional role during mitosis, underlining CRM1's relevance as a mitotic effector (Knauer et al., 2006).

In addition, NES-mediated nuclear export of regulatory viral proteins is mandatory for their biological activity and viral replication, and hence, for disease development and progression. Such proteins include the Human Immunodeficiency Virus Type 1 (HIV-1) Rev protein, required for the transport of unspliced and incompletely spliced viral RNAs from the nucleus to the cytoplasm (Stauber et al., 1995). As such, Rev's export function is crucial for the generation of infectious viral particles and ultimately for development of AIDS (Pavlakis and Stauber, 1998; Stauber et al., 1995). As not only complex retroviruses but also human pathogenic DNA viruses, such as adeno- and herpesviruses, depend on NES-mediated export, targeting transport by molecular decoys appears to be an attractive therapeutic strategy (Kratzer et al., 2000).

The role of CRM1 as an export receptor and a mitotic effector was first elucidated through the use of the cytotoxic polyketide leptomycin B (LMB), which covalently inhibits CRM1 through a Michael addition of LMB onto Cys528. A similar mode of action was identified for other CRM1 inhibitors, such as LMB derivates, Ratjadones or N-azolylacrylates (Meissner et al., 2004; Mutka et al., 2009; Van Neck et al., 2008; US patents: US7,446,196 B2; US2005/0203174 A1). LMB's clinical development was discontinued due to the significant toxicity observed without apparent efficacy (Mutka et al., 2009; Newlands et al., 1996). Notwithstanding LMB's initial failure in the clinic, nuclear export inhibitors may represent a novel class of anti-virals and cancer therapeutics. Such compounds would *inter alia* derive their activity by preventing cytoplasmic localization and inactivation of important tumor suppressors that are dependent on CRM1 for nuclear export, such as p53 (Brown et al., 2009). It has been estimated that roughly 50% of all tumors, including cancers of the head and neck, maintain wild-type p53 (Brown et al., 2009; Poeta et al., 2007). In many of these cases, the tumor suppressor function is compromised by overexpression or inactivation of cellular factors that regulate the levels of p53 in the nucleus or lead to its enhanced export out of the nucleus (Brown et al., 2009; Poeta et al., 2007). Also, in human papilloma virus (HPV)-positive cancers, aberrant cytoplasmic localization and/or degradation of p53 prevents the activation of pathways that would lead to cell death (Brown et al., 2009). Consequently, a means of relocalizing the anti-oncogenic wild-type p53 to the nucleus in these aberrant cell types is a promising approach to regaining control of cell proliferation and apoptosis (Brown et al., 2009).

The problem underlying the invention is to provide compounds which inhibit nuclear export of antiviral and tumor-suppressing cellular factors and which are therefore suitable for use in the treatment or prophylaxis of cancer or viral diseases.

The invention relates to a Compound of formula I or a pharmaceutically acceptable salt, solvate, hydrate or N-oxide thereof for use in the treatment or prophylaxis of cancer or viral diseases 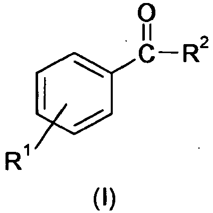 wherein
R¹ is halogen or halo-C₁-C₆-alkyl;
R² is a group of formula II or III; 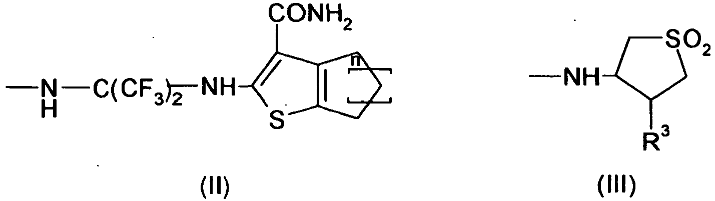 and
R³ is halogen, and
n is 1 or 2.

Preferably, R³ is chlorine.

Further, the invention relates to a pharmaceutical composition comprising at least one compound of formula I or a pharmaceutically acceptable salt, solvate, hydrate or N-oxide thereof.

According to an embodiment, R¹ is chlorine, fluorine or trifluoromethyl.

A compound of formula Ia or Ib is preferred: 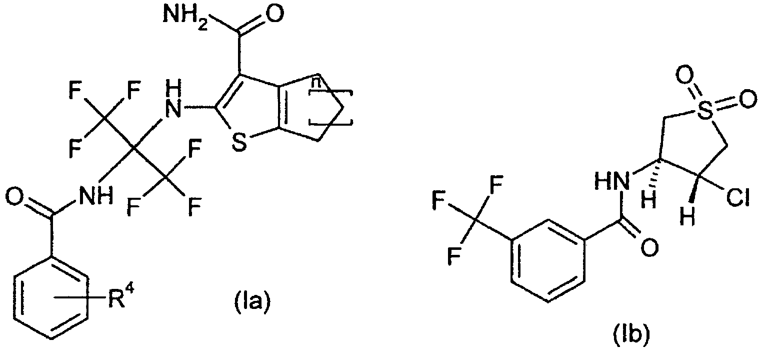
wherein R⁴ is preferably fluorine or chlorine,
and n is 1 or 2.

Preferred compounds of formula la are 2-[[1,1,1,3,3,3-hexafluoro-2-[(2-fluorobenzoyl)amino]propan-2-yl]amino]-4,5,6,7-tetrahydro-1-benzothiophene-3-carboxamide, 2-[[1,1,1,3,3,3-hexafluoro-2-[(3-fluorobenzoyl)amino]propan-2-yl]amino]-5,6-dihydro-4H-cyclopenta[b]thiophene-3-carboxamide and 2-[[1,1,1,3,3,3-hexafluoro-2-[(3-chlorobenzoyl)amino]propan-2-yl]amino]-4,5,6,7-tetrahydro-1-benzothiophene-3-carboxamide. The IUPAC name of the compound of formula Ib is N-(4-chloro-1,1-dioxothiolan-3-yl)-3-(trifluoromethyl)benzamide.

It is assumed that the compounds of the invention inhibit nuclear export of proteins, such as Survivin, Ad 5 E1B-55K, B23, Bcr-Abl, HIV-1 Rev, p53, p73, STAT1, ADAR1 and actin, by blocking the CRMI-dependent export of these proteins. The compounds of the invention may act by non-covalent binding of CRM1.

The cancer is in particular selected from the group consisting of fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovialoma, mesothelioma, epidermoid carcinoma, Ewing tumor, leiosarcoma, rhabdomyosarcoma, colon carcinoma including colon adenocarcinoma, pancreas cancer, head and neck cancer, mamma carcinoma, ovarial cancer, prostate carcinoma, squamous cell carcinoma, basal cell carcinoma, adenocarcinoma, syringoma, sebaceous carcinoma, papillary carcinoma, papillary adenocarcinoma, cystadenocarcinoma, bone marrow carcinoma, bronchogenic carcinoma, renal cell carcinoma, hepatoma, cholangioma, chorionepithelioma, seminoma, embryo carcinoma, Wilms' tumor, cervix carcinoma, HPV-associated cervix carcinoma, testicular cancer, lung carcinoma, small-cell lung carcinoma, bladder carcinoma, epithelial carcinoma, glioma, astrocytoma, medulloblastoma, craniopharyngioma, ependymoma, pinealoma, haemangioblastoma, acustic neuroma, oligodendroglioma, meningioma, melanoma, neuroblastoma, retinoblastoma, leukemia such as chronic myeloid leukemia (CML), acute lymphoblastic leukemia (ALL) such as B cell precursor ALL, acute myeloid leukemia (AML), chronic lymphoid leukemia (CLL) and other lymphomas, multiple myeloma, Waldenstroem's macroglobulinemia and heavy-chain disease.

The pathogen of the viral diseases is for example selected from the group consisting of adenoviridae such as adenovirus; picornaviridae such as coxsackievirus, hepatitis A virus, poliovirus, rhinovirus and echovirus; herpesviridae such as herpes simplex type 1 (HSV-I), herpes simplex type 2 (HSV-II), Varicella-zoster virus, Epstein-barr virus, human cytomegalovirus, human herpesvirus type 8; hepadnaviridae such as hepatitis B virus; flaviviridae such as hepatitis C virus, West Nile virus, dengue virus, yellow fever virus; retroviridae such as human immune deficiency virus type I (HIV-I), human immune deficiency virus type II (HIV-II) and human T-lymphotropic virus (HTLV); orthomyxoviridae such as influenza virus; paramyxoviridae such as measles virus, mumps virus, parainfluenza virus, respiratory syncytial virus, human metapneumovirus and morbillivirus; papillomaviridae such as papillomavirus; reoviridae such as rotavirus; rhabdoviridae such as rabies virus; togaviridae such as rubella virus; polyomaviridae such as papovavirus; bunyaviridae such as hantaviruses; and parvoviridae such as human bocavirus and parvovirus.

The compounds of formula I can be used in their neutral form. The present invention further comprises the use of said compounds in the form of pharmaceutically acceptable salts which are derived from organic and inorganic acids and bases. Suitable bases are, for example, alkali metal hydroxides such as potassium hydroxide, sodium hydroxide and lithium hydroxide; alkaline earth metal hydroxides such as calcium hydroxide; alkali metal alcoholates such as potassium ethanolate and sodium propanolate; as well as various organic bases such as piperidine, diethanolamine, N-methylglutamine, N,N'-dibenzylethylenediamine, chlorprocaine, choline, diethanolamine, ethylenediamine, N-methyl'D-glucamine and procain. Suitable pharmaceutically acceptable acid addition salts of the compounds of the invention are: acetate, adipate, alginate, arginate, aspartate, benzoate, benzenesulfonate (besylate), bisulfate, bisulfite, bromide, butyrate, camphorate, camphor sulfonate, caprylate, chloride, chlorobenzoate, citrate, cyclopentanpropionate, digluconate, dihydrogenphosphate, dinitrobenzoate, dodecylsulfate, ethanesulfonate, fumarate, galactarate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, or phthalate.

Suitable derivatives of the compounds of formula I are for example esters, amides, N-oxides and the like. Further, prodrugs are to be mentioned as derivatives. Prodrugs are compounds that are metabolized *in vivo* to the compounds of formula I. Typical examples of prodrugs are disclosed in C.G. Wermeth (ed.): The Practice of Medicinal Chemistry, Academic Press, San Diego, 1996, pages 671-715. These include, for example, phosphates, carbamates, amino acids, esters, amides, peptides, ureas and the like.

The present invention includes the stereoisomers and each individual (R) und (S) enantiomer of the compounds essentially free of, i.e. containing less than 5%, preferably less than 2%, in particular less than 1% of the other enantiomers and a mixture of such enantiomers in any ratio, including racemic forms that contain practically the same proportions of two enantiomers.

For the preparation of a pharmaceutical composition the compounds of formula I can be formulated together with at least one solid, liquid and/or semiliquid carrier or excipient and optionally together with one or more additional active compounds to a suitable dosage form. The compositions can be obtained by using a process which is generally known in the pharmaceutical field.

Pharmaceutical compositions can be administered in the form of dosage units which contain a predetermined amount of active ingredient per dosage unit. Such unit may contain for example 0.1 mg to 3 g, preferably 1 mg to 700 mg, especially preferred 5 mg to 100 mg of a compound of the invention depending on the disorder to be treated, the route of administration and the age, sex, weight and condition of the patient.

Pharmaceutical compositions can be adapted for administration on any suitable route, for example on the oral (including buccal or sublingual), rectal, nasal, topical (including buccal, sublingual or transdermal), vaginal or parenteral (including subcutaneous, intramuscular, intravenous or intradermal). Such formulations can be obtained by using any process known in the pharmaceutical field by combining for example the active compound with the carrier(s) or excipient(s).

Pharmaceutical formulations for oral administration can be provided as separate units such as tablets, capsules or nanocarriers; powders or granules; solutions or suspensions in aqueous or non-aqueous liquids; or oil-in-water or water-in-oil suspensions.

According to another embodiment of the invention the compound of formula I is administered in combination with at least one additional anti-cancer agent.

The additional anti-cancer agent is for example selected from the group consisting of topoisomerase I inhibitors (such as irinotecan, topotecan, and camptothecin); topoisomerase II inhibitor (such as etoposide, teniposide, doxorubicin, epirubicin, idarubicin, mitoxantrone and daunorubicin); alkylating agents (such as mechlorethamine, estramustine, uramustine, melphalan, chlorambucil, busulfan, thiotepa and its analogues, ifosfamid, carmustine, lomustine, semustine, streptozocin, dacarbazine, temozolomide, mitomycin C, procarbazine, altretamine and cyclophosphamide); platinum analogues (such as cisplatin, nedaplatin, satraplatin, triplatin tetranitrate, oxaliplatin and carboplatin); antimetabolites such as methotrexat and nucleoside mimetics (such as 5fluorouracil, capecitibin, gemcitabin, fludarabin, cytarabin, mercaptopurine, azathioprine, thioguanine, pentostatin and hydroxyurea), taxanes (such as paclitaxel and docetaxel); epothilones (such as ixabepilone); vinca alkaloids (such as vincristine and vinblastine); tyrosine kinase inhibitors (such as imatinib, dasatinib, nilotinib and gefitinib); proteasome inhibitors (such as bortezomib); NF-κB-Inhibitors, including inhibitors of IκB kinase; antibodies, which bond to proteins expressed by cancer cells and inhibit cell proliferation or/and angiogenesis (such as trastuzumab, rituximab, cetuximab and bevacizumab).

In another embodiment of the invention the compound of formula I is administered in combination with at least one additional antiviral agent.

The additional antiviral agent is for example selected from the group consisting of nucleoside reverse transkriptase inhibitors (NRTI) (such as zidovudine (AZT), didanosine ("DDI"), zalcitabine ("DDC"), stavudine (d4T), ribavirin, lamivudine (3TC), abacavir (ABC), emtricitabine (FTC), entecavir (INN), Apricitabine (ATC), tenofovir, adefovir,), non-nucleoside reverse transkriptase inhibitors (NNRTI) (such as nevirapine, delaviridine, efavirenz, etravirine), protease inhibitors (such as saquinavir, ritonavir, indinavir, lopinavir, atazanavir, fosamprenavir, tipranavir, darunavir, telinavir, amprenavir and nelfinavir), integrase inhibitors (such as AR177), castanospermin, 2-deoxy-D-glucose ("2-dGlc"), deoxynojirimycin, acycloguanosin, rifampicin, adamantidine, rifabutin, ganciclovir ("DHPG"), fluoroiodoaracytosine, idoxurin, trifluorothymidine, adeninarabinoside ("ara-A"), ara-AMP, bromovinyldeoxyuridine, bromovinylarauracil, rimantadin, arildone, diarylamidine, (S)-(p-Nitrobenzyl-)6-thioinosine, inhibitors of pyrophosphorolysis and/or inhibitors of the ribonucleotide dependent phosphorolyis. The additional antiviral agent can also be selected from cytokine or cytokine inhibitors, such as rIFN-alpha, rIFN-beta, rIFN-gamma, inhibitors of TNF-alpha and MNX-160.

The compound of formula I and the additional anti-cancer agent and/or the additional antiviral agent can be administered separately or together, simultaneously or sequentially, for example as a co-formulation or as separate formulations.

The following examples and figures illustrate the invention.

The following abbreviations are used in the examples and figures:

| Compound | IUPAC-name | CAS Registry Number |
|---|---|---|
| C3 | 2-[[1,1,1,3,3,3-hexafluoro-2-[(2-fluorobenzoyl)amino]propan-2-yl]amino]-4,5,6,7-tetrahydro-1-benzothiophene-3-carboxamide | 352317-01-2 |
| C5 | N-(4-chloro-1,1-dioxothiolan-3-yl)-3-(trifluoromethyl)benzamide | 440662-95-3 |
| C24 | 2-[[1,1,1,3,3,3-hexafluoro-2-[(3-fluorobenzoyl)amino]propan-2-yl]amino]-5,6-dihydro-4H-cyclopenta[b]thiophene-3-carboxamide | 361367-01-3 |
| C28 | 2-[[1,1,1,3,3,3-hexafluoro-2-[(3-chlorobenzoyl)aminolpropan-2-yl]amino]-4,5,6,7-tetrahydro-1-benzothiophene-3-carboxamide | 309731-52-0 |

### Figures

Figure 1 shows that nuclear export of Survivin and of the HIV-1 Rev protein is inhibited following treatment with 25µM C3 or C5 (Example 1.2).
Figure 2 shows the viability of different cancer cell lines after treatment with 100µM C3 or C5 for 72h (Example 2). Columns show means and bars show ± standard deviations of three independent experiments.
Figure 3 shows the viability of K562 cells after treatment with imatinib, C3 or C5, or combinations of imatinib together with C3 or C5 for 72h (Example 3). Columns show means and bars show ± standard deviations of three independent experiments.
Figure 4 shows the viability of HeLa cells and primary non-malignant human fibroblasts (HTF-8) 24h after treatment with 100µM C3 or C5 for 72h (Example 4). Columns show means and bars show ± standard deviations of three independent experiments.
Figure 5 shows the body weights of six-week-old male nude mice, that were injected intraperitoneally (i.p.) on days 1, 3 and 5 with 200µl 1mM C3 or C5, or with DMSO only (Example 5). 5 mice were treated with C3, 5 mice were treated with C5 and 2 mice were treated with DMSO only (control).
Figure 6 shows the results of an HIV-1 replication inhibition assay using HIV-1 infected Tzm-bl cells and C3 or C5, respectively (Example 6). A: Experimental setting. Tzm-bl cells were infected with HIV-1. 24h post infection, 25 or 50µM C3 or C5 were added. 48h post infection reverse transcriptase (RT) activity was analyzed in the viral supernatant. B: RT-activity measured 24h after addition of 25 or 50µM C3 or C5 or DMSO only (control).
Figure 7 shows the immunoblot analysis of eluates obtained from beads with immobilized recombinant GST-Survivin_{NES}-GFP that were used for pulling down Crm1-HA from samples preincubated with DMSO only, LMB, C3 or C5 (Example 7). The proteins bound to the beads, i.e. GST-Survivin_{NES}-GFP and the Crm1-HA bound to it, were eluted, and the eluates analyzed after SDS-gelelectrophoresis by immunoblot using HA specific antibodies for detecting Crm1-HA and GFP specific antibodies for detecting GST-Survivin_{NES}-GFP (loading control).

### Examples

### Example 1: C3, C5 and C24 inhibit NES-dependent translocation of biosensors in living cancer cells

### Example 1.1: Inhibition of translocation of Rev_{NES}-biosensor

The activity of compounds C3, C5 and C24 to inhibit NES-mediated nuclear export in living cells was analyzed as described in Fetz et al., 2009 using living HeLa (cervix carcinoma), FaDu (head and neck cancer), MDA-MB231 (mammacarcinoma) and SW480 cells (colon cancer) which transiently expressed the Rev_{NES}-biosensor. The Rev_{NES}-biosensor is a fusion protein composed of (from N to C terminus) the SV40 large T-antigen nuclear localization signal (NLS), glutathion-S-transferase (GST), green fluorescent protein (GFP) and the nuclear export signal (NES) of the HIV-1 (human immunodeficiency virus) Rev protein (Rev_{NES}).

### a) Time-dependent inhibition of nuclear export in Rev_{NES}-biosensor expressing HeLa cells

C3, C5 or C24 (stock solution in DMSO) was added to living HeLa cells transiently expressing Rev_{NES}-biosensor to a final concentration of 25µM. The cells of the control were treated with a corresponding amount of DMSO only. The cellular localization of Rev_{NES}-biosensor was monitored by fluorescence microscopy.
In the cells of the control the Rev_{NES}-biosensor was predominantly localized in the cytoplasm. In contrast, treatment with compounds C3, C5 or C24 resulted in nuclear accumulation of the biosensor indicating blockage of nuclear export. Maximum of cellular accumulation was observed after 1h (C24), after 2h (C3) and after 5h (C5), respectively. After 24h C5 treated cells still showed nuclear fluorescence, whereas in C3 and C24 treated cells fluorescence was predominantly localized in the cytoplasm. Thus, the inhibitory activity of C3 and C24 (reversible) but not of C5 (irreversible) was reversed after 24h.

**Table 1: Cellular localization of green fluorescent Rev_{NES}-biosensor**

| | control | C3 (Tₒₚₜ = 2h) | C5 (Tₒₚₜ = 5h) | C24 (Tₒₚₜ = 1h) |
|---|---|---|---|---|
| Tₒₚₜ* | cytoplasm | nucleus | nucleus | nucleus |
| T = 24h | cytoplasm | cytoplasm | nucleus | cytoplasm |

| | | | | |
|---|---|---|---|---|
| * Tₒₚₜ = time point post treatment when maximum export inhibition is observed | | | | |

### b) Time-dependent inhibition of nuclear export in Rev_{NES}-biosensor expressing FaDu, MDA-MB231 and SW480 cells

Similar to what was observed with HeLa cells, nuclear accumulation of the Rev_{NES}-biosensorwas also observed in biosensor-expressing FaDu, MDA-MB231 and SW480 cells treated with 25µM C3 or C5. Maximum nuclear localization of fluorescence was observed after 2h (C3) and 5h (C5), respectively.

**Table 2: Cellular localization of green fluorescent Rev_{NES}-biosensor**

| | Control | C3 (Tₒₚₜ = 2h) | C5 (Tₒₚₜ = 5h) |
|---|---|---|---|
| FaDu | cytoplasm | nucleus | nucleus |
| MDA-MB231 | cytoplasm | nucleus | nucleus |
| SW480 | cytoplasm | nucleus | nucleus |

| | | | |
|---|---|---|---|
| * Tₒₚₜ = time point post treatment when maximum export inhibition is observed | | | |

### c) Concentration-dependent inhibition of nuclear export in Rev_{NES}-biosensor expressing HeLa cells

**Table 3: Cellular localization of green fluorescent Rev_{NES}-biosensor**

| | C3 | | C5 | |
|---|---|---|---|---|
| concentration | T = 0h | T = 1h | T = 0h | T = 3h |
| 25µM | cytoplasm | nucleus/cytoplasm | cytoplasm | nucleus/cytoplasm |
| 75µM | cytoplasm | nucleus | cytoplasm | nucleus |
| 150µM | cytoplasm | nucleus | cytoplasm | nucleus |

### Example 1.2: Inhibition of nuclear export of Survivin and HIV-1 Rev protein

The activity of compounds to inhibit the export of the oncologically relevant protein Survivin as well as of the HIV-1 Rev protein was analyzed.

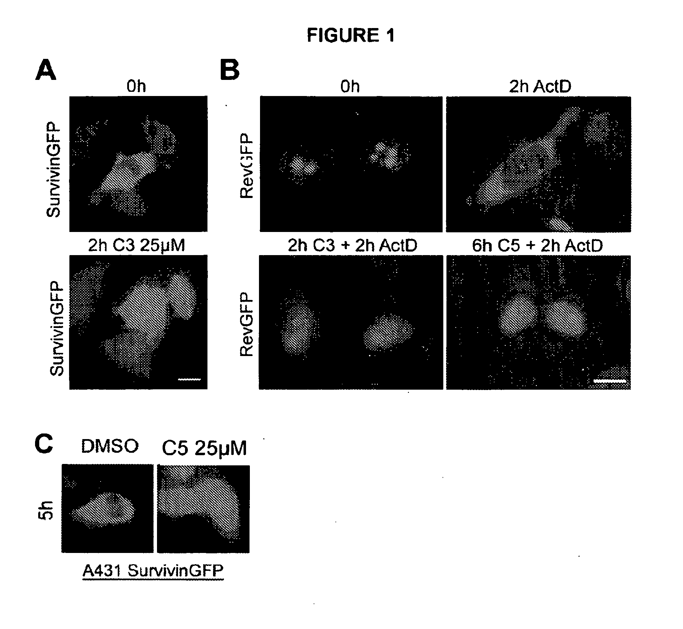

Nuclear export of Survivin is crucial for its tumor promoting cytoprotective activity (Engels et al., 2007; Knauer et al., 2007a; Knauer et al., 2007b). Transient expression of a Survivin-GFP fusion in HeLa cells resulted in a cytoplasmic protein (Knauer et al., 2007b), which accumulated in the nucleus upon treatment with 25µM C3, C5 and C24 (Fig. 1A). Likewise, treatment with 25µM C5 caused nuclear accumulation of an otherwise cytoplasmic Survivin-GFP fusion that was stably expressed in A431 breast cancer cells (Fig. 1 C).

The HIV-1 Rev protein transports unspliced and incompletely spliced viral RNAs from the nucleus into the cytoplasm (Stauber et al., 1995). As such, Rev's export function is crucial for viral replication and AIDS development (Pavlakis and Stauber, 1998; Stauber et al., 1995). To demonstrate export inhibition of the biologically active HIV-1 Rev-GFP protein, HeLa cells transiently expressing the nuclear/nucleolar fusion protein were treated with actinomycin D (ActD), which affected Rev-GFP's nucleolar accumulation, promoting its nuclear export and subsequent cytoplasmic localization. Inhibition of export by treatment with 25µM C3, C5 or C24 restored the nuclear accumulation of Rev-GFP (Fig. 1B).

### Example 2: C3, C5 and C24 prevent proliferation and induce apoptosis of adherent und non-adherent cancer cell lines

To test the anti-cancer activity of the compounds, several cancer cell lines, currently used as models for different epithelial cancers and leukemias, were treated with the indicated inhibitors. Indicated cell lines were seeded at a density of 10³ cells/well in 96 well plates, and treated with the compounds (stock solution in DMSO, final concentration in medium: 100µM) 24h later. Every 24h, the cell culture medium was replaced by fresh compound-containing medium. 72h post treatment, cell were analyzed by adding 100µl CellTiter-Glo® assay reagent (Promega). Luminescence was measured with a LuminoskanAscent device (ThermoScientific). Cells treated with a corresponding amount of DMSO only were use as a control. The luminescence (equals cell viability) of said control was set to 100%.

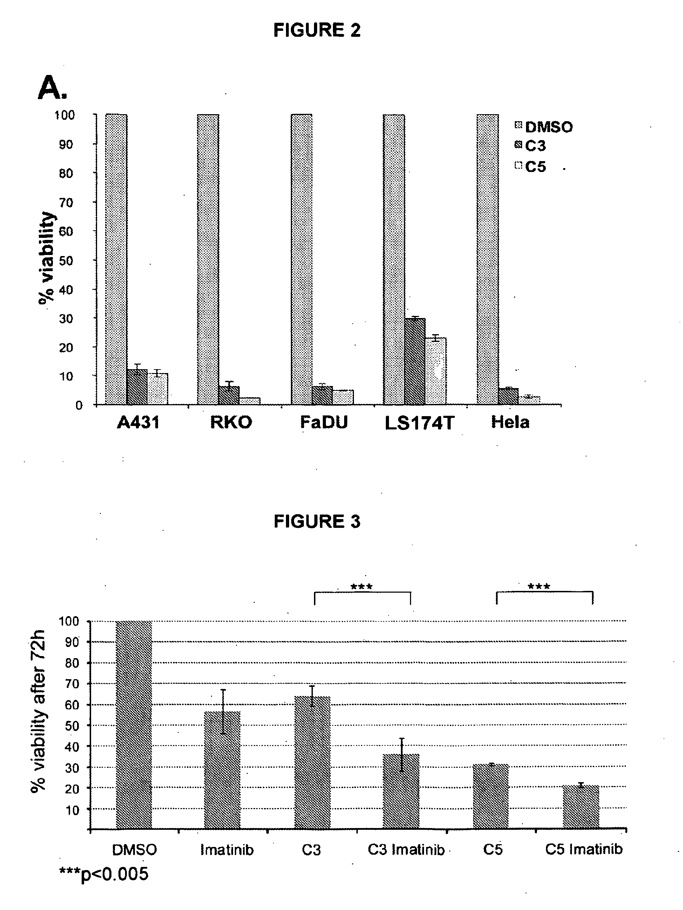

A431, RKO, FaDu, LS173T and HeLa cells treated with C3 or C5 showed significantly reduced viability compared to the control (see Figure 2). Further, cell death induced by treatment with 25µM C3 or C5 was observed in HeLa (cervix carcinoma), FaDu (head and neck cancer), RKO (colon cancer), A431 (epidermoid carcinoma), LS174T (colon adenocarcinoma), K562 (Bcr-Abl⁺) (chronic myeloid leukemia, CML), SUP-B15 (acute lyphoblastic leukemia, B cell precursor ALL) and KASUMI-1 (acute myeloid leukemia, AML) but not in HTF-8 (primary human, non-malignant fibroblast) cells.

Further, drug-induced apoptosis was shown by immunoblot detection of cleaved Caspase 3 and PARP cleavage (α-cleaved Caspase 3 CellSignaling, 9661; α-PARP Ab: BD Pharmingen, 556362) in lysates prepared 72h post treatment from HeLa cells treated with 25µM C3 or C5. Lysates of cells treated DMSO only were used as a control.

The functional characteristics of compounds C3, C5 and C24 are summarized in Table 4. IC₅₀ value corresponds to compound concentration reducing HeLa cell viability by 50%. Tₒₚₜ is the time point post treatment when maximum nuclear export inhibition was observed by fluorescence microscopy.

**Table 4: Functional characteristics of nuclear export inhibitors**

| Compound | Mode of Inhibition | IC₅₀ | Tₒₚₜ at 25µM |
|---|---|---|---|
| C3 | reversible | 38µM | ∼1h |
| C5 | irreversible* | 17µM | 5-6h |
| C24 | reversible | 31µM | ∼1h |

| | | | |
|---|---|---|---|
| *5-12h observation | | | |

### Example 3: Nuclear export inhibitors and chemotherapeutics synergize in the killing of cancer cells

The BCR-ABL⁺ human chronic myeloid leukemia (CML) cell line K562 was used to investigate whether combining export inhibitors with chemotherapeutic drugs currently used in the clinics enhances drug-induced cancer cell death. AlamarBlue® viability assays revealed that the BCR-ABL tyrosine kinase inhibitor imatinib or C3 and C5 individually caused cancer cell death, however, co-administration of imatinib with C3 or C5 was significantly more effective by comparison (Figure 3).

For AlamarBlue® viability assays 3x10⁵ K562 cells/well were seeded in 96 well plates and treated with the indicated drugs (stock solution in DMSO, final concentration in cell culture medium: C3/C5 = 25µM, imatinib = 100nM). Every 24h nuclear export inhibitors and imatinib were added at the indicated concentrations. 72h post treatment, cells were analyzed by adding 10µl of AlamarBlue® (Invitrogen). Fluorescence (538/600nm) was measured with a LuminoskanAscent (ThermoScientific). Cells treated with DMSO only were used as a control. The fluorescence measured (equals cell viability) with said control was set to 100%. Export inhibitors were observed to synergize with imatinib in the killing of CML cells.

### Example 4: C3, C5 and C24 do not affect viability of non-malignant cells

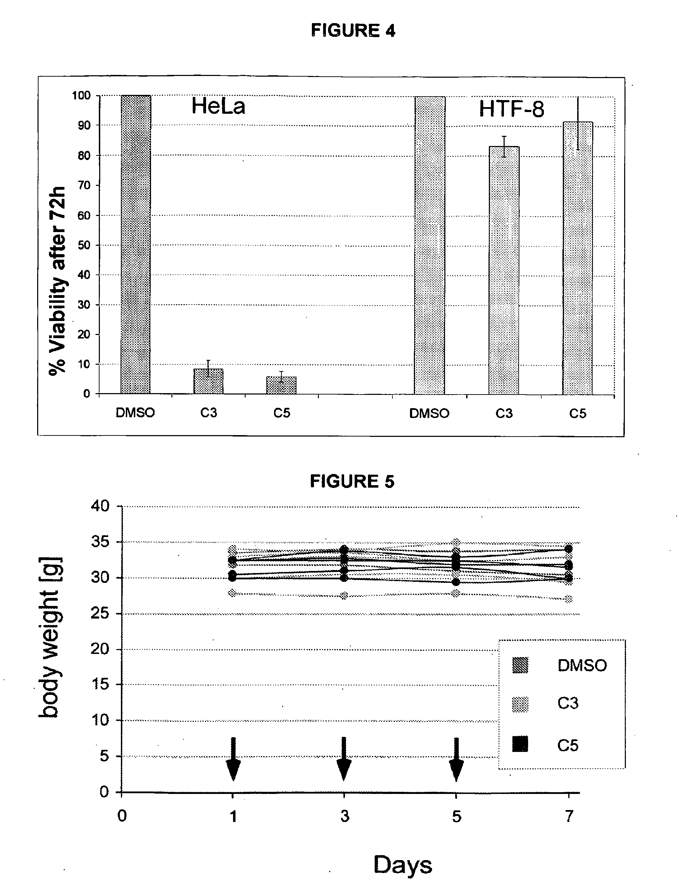

It was assessed whether the export inhibitors also affect the viability of non-malignant human cells, precluding their potential (pre)clinical use. 10³ freshly isolated non-malignant primary human fibroblasts (HTF-8) or HeLa cells/well were seeded and 24h later treated with the indicated compounds (final concentration: 100µM). Every 24h cell culture medium was replaced by freshly prepared compound-containing medium. 72h post treatment the cells were analyzed by adding 100µl CellTiter-Glo® assay reagent, and luminescence measured with a LuminoskanAscent (ThermoScientific). Cells treated with DMSO only were used as a control. The fluorescence measured (equals cell viability) with said control was set to 100%. C3 and C5 were shown to significantly affect the viability of HeLa cancer cells but not of non-malignant fibroblasts (Figure 4).

### Example 5: C3 or C5 do not induce cytotoxicity in nude mice

To investigate whether the nuclear export inhibitors cause toxic effects *in vivo,* six-week-old male NMRI *nu*/*nu* mice were injected three times intraperitoneally (i.p.) every second day with 200µl 1mM C3 or C5 (5 mice each), or with 200µl DMSO only (control, 2 mice). Compared to the DMSO control, no significant weight loss (see Figure 5) or other signs of *in vivo* toxicity were observed.

### Example 6: C3 and C5 inhibit HIV-1 replication

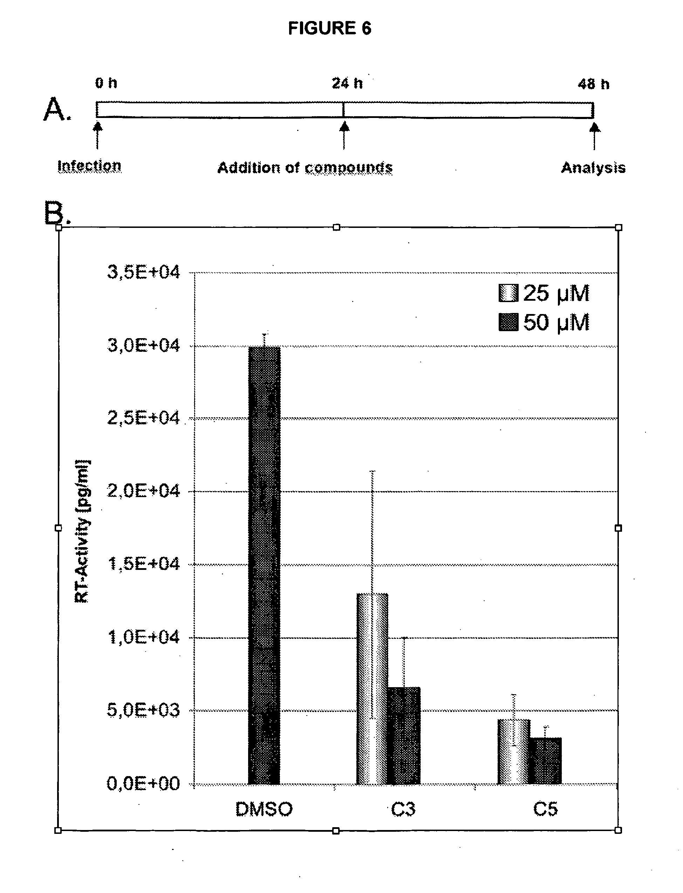

The HIV-1 Rev protein transports unspliced and inclompletely spliced viral RNAs from the nucleus into the cytoplasm, crucial for viral replication and AIDS development (Pavlakis and Stauber, 1998; Stauber et al., 1995). To directly demonstrate that C3 and C5 are able to inhibit viral replication, 2.5x10⁵ Tzm-bl cells (HeLa derived cells expressing the HIV-1 (co)receptors CD4, CXCR4 and CCR5; see Montefiori, 2009) were infected with HIV-1 (strain NL4-3; CXCR-4 tropic; 20 000pg Reverse Transcriptase Units). 24h post infection, DMSO only (control) or compounds C3 or C5 (stock solution in DMSO) were added to a final concentration of 25 or 50µM. 24h later (= 48h post infection) the viral particles in the supernatant were quantified by measuring reverse transcriptase (RT) activity in the viral supernatant using the RT-Lenti Kit (Cavidi-Tech; Uppsala, Sweden) (see Fig. 6A).

As shown in Fig. 6B, compared to the control treatment with compounds C3 and C5 significantly reduced the RT-activity, i.e. inhibited the production of viral particles, in a dose-dependent manner, probably by inhibition of the nuclear export of the HIV-1 Rev protein. These results demonstrate that C3 and C5 are expected to show anti-viral efficacy also in a pathophysiological setting, i.e., in humans, monkeys or mouse models.

### Example 7: Nuclear export inhibition by C3 and C5 does not rely on covalent binding to CRM1

The binding of LMB and of compounds C3 and C5 to CRM1 was examined by *in vitro* pull-down assays as described by Stauber et al., 2006. Briefly, *in vitro* coupled transcription/translation was performed using the TNT reticulocyte lysate system (Promega, Madison, WI, USA) supplemented with methionine (Promega, Madison, WI, USA) and plasmid pCrm1_HA (1µg), encoding a hemagglutinin epitope-tagged version of human CRM1 (Crm1-HA), as the template. Equal amounts of the Crm1-HA TNT reaction were preincubated with either DMSO (negative control; 0.1%), LMB (400nM, final concentration), C3 (1 mM; final concentration) or C5 (1mM; final concentration) in binding-buffer containing 5mM MgCl₂ (AppliChem, Darmstadt, Germany), 2mM GTP (PEQLAB, Erlangen, Germany), 50mM Hepes pH 7.9 (AppliChem, Darmstadt, Germany), 200mM KCI (AppliChem, Darmstadt, Germany), 0.4% Tween 20 (AppliChem, Darmstadt, Germany), supplemented with complete protease inhibitor cocktail (Roche, Mannheim, Germany) for 30min at 4°C. Subsequently, GST-Sepharose beads loaded with 25µg recombinant GST-Survivin_{NES}-GFP and 5µg GST-RanQ96L were added to each drug-treated TNT reaction. Recombinant GST-Survivin_{NES}-GFP and GST-RanQ96L were purified from bacteria as described by Stauber et al., 2006. Pull-down was performed for 4h at 4°C in a rotating wheel. Samples were then centrifuged (3500rpm, 1min, 4°C) to recover the GST-Survivin_{NES}-GFP beads with bound Crm1-HA. Beads were washed three times with binding-buffer (500µl each washing step, 3500rpm, 1min, 4°C). Finally, bound proteins were eluted by adding 25µl 2xSDS-sample buffer (250mM Tris-HCl pH 6.8; 2% w/v SDS, 20% glycerol, 2% β-mercaptoethanol, 0.002% w/v bromophenol blue) and incubating at 95°C for 2min. Samples were separated by SDS-gelelectrophoresis and analyzed by immunoblot analysis as described by Stauber et al., 2006. Crm1-HA was detected with HA specific antibodies (MMS-101R, Covance, New Jersey, USA). GFP served as the loading control and was visualized with GFP specific antibodies (sc-8334, Santa Cruz Biotechnology, Heidelberg, Germany).

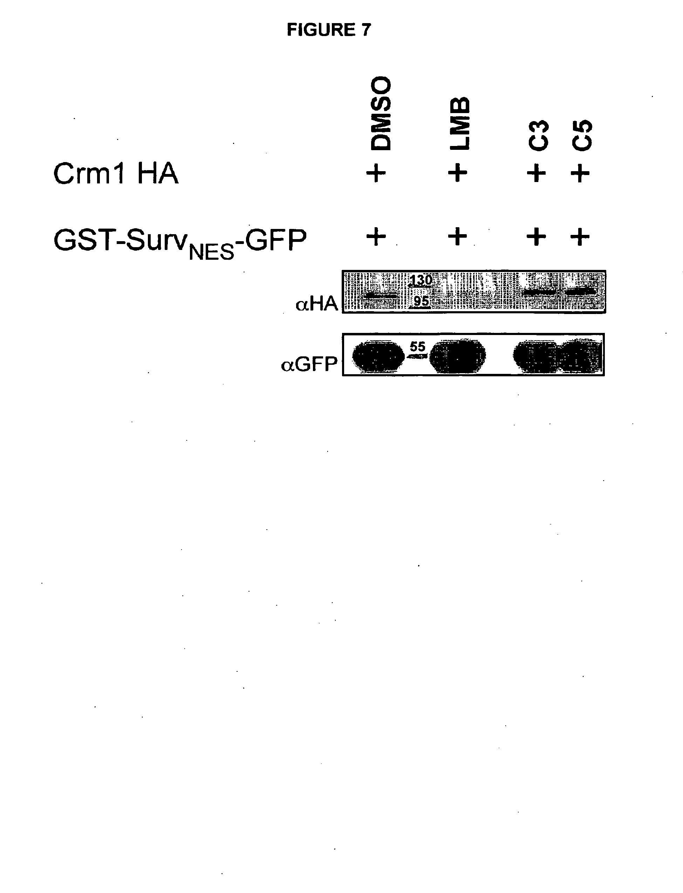

As shown in Fig. 7, the covalent binding of LMB to Crm1-HA prevented the interaction of Crm1-HA with the recombinant export substrate GST-Survivin_{NES}-GFP. In contrast, pretreatment with C3, C5 or DMSO alone did not affected the CRM1 binding to the export substrate. These results indicate that compounds C3 and C5 do not covalently bind to Crm1-HA and act *via* a molecular mechanism distinct from that of LMB.

### Literature

Brown, C.J., Lain, S., Verma, C.S., Fershi, A.R., Lane, D.P., 2009. Awakening guardian angels: drugging the p53 pathway. Nat Rev Cancer 9, 862-873.
Cullen, B.R., 2003. Nuclear RNA export. J Cell Sci 116, 587-597.
D'Angelo, M.A., Raices, M., Panowski, S.H., Hetzer, M.W., 2009. Age-dependent deterioration of nuclear pore complexes causes a lot of nuclear integrity in postmitotic cells. Cell 136, 284-295.
Engels, K., Knauer, S.K., Metzler, D., Simf, C. Struschka, O., Bier, C., Mann, W., Kovacs, A.F., Stauber, R.H., 2007. Dynamic intracellular Survivin in oral squamous cell carcinoma: underlying molecular mechanism and potential as an early prognostic marker. J Pathol 211, 532-540.
Fahrenkrog, B., Aebi, U., 2003. The nuclear pore complex: nucleocytoplasmic transport and beyond. Nat Rev Mol Cell Biol 4, 757-766.
Faustino, R.S., Nelson, T.J., Terzic, A., Perez-Terzic, C., 2007. Nuclear transport: target for therapy. Clinical pharmacology and therapeutics 81, 880-886.
Fetz, V., Knauer, S.K., Bier, C., Kriess, J.P., Stauber, R.H., 2009. Translocation Biosensors - Cellular System Integrators to Dissect CRM1-Dependent Nuclear Export by Chemicogenomics. Sensors 7(9), 5423-5445.
Hurt, J.A., Silver, P.A., 2008. mRNA nuclear export and human disease. Dis Model Mech 1, 103-108.
Kau, T.R., Way, J.C., Silver, P.A., 2004. Nuclear transport and cancer: from mechanism to intervention. Nat Rev Cancer 4, 106-117.
Knauer, S.K., Bier, C., Habtemichael, N., Stauber, R.H., 2006. The Survivin-Crm1 interaction is essential for chromosomal passenger complex localization and function. EMBO Rep 7, 1259-1265.
Knauer, S.K., Bier, C., Schlag, P., Fritzmann, J., Dietmaier, W., Rodel, F., Klein-Hitpass, L., Kovacs, A.F., Doring, C., Hansmann, M.L., Hofmann, W.K., Kunkel, M., Brochhausen, C., Engels, K., Lippert, B.M., Mann, W., Stauber, R.H., 2007a. The Survivin-3B is cytoprotective and can function as a chromosomal passenger complex protein. Cell Cycle 6, 1502-1509.
Knauer, S.K., Kramer, O.H., Knosel, T., Engels, K., Rodel, F., Kovacs, A.F., Dietmaier, W., Klein-Hitpass, L., Habtemichael, N., Schweitzer, A., Brieger, J., Rodel, C., Mann, W., Petersen, I., Heinzel., T., Stauber, R.H., 2007b. Nuclear export is essential for the tumor-promoting activity of Survivin. Faseb J 21, 207-216.
Knauer, S.K., Moodt, S., Berg, T., Liebel, U., Pepperkok, R., Stauber, R.H., 2005. Translocation biosensors to study signal-specific nucleo-cytoplasmic transport, protease activity and protein-protein interactions. Traffic (Copenhagen, Denmark) 6, 594-606.
Kramer, O.H., Knauer, S.K., Greiner, G., Jandt, E., Reichart, S., Guhrs, K.H., Stauber, R.H., Bohmer, F.D., Heinzel, T., 2009. A phosphorylation-acetylation switch regulates STAT1 signaling. Genes Dev 23, 223-225.
Krätzer, F., Rosorius, O., Helger, P., Hirschmann, N., Dobner, T., Hauber, J., Stauber, R.H., 2000. The adenovirus type 5 E1B-55K oncoprotein is a highly active shuttle protein and shuttling is independent of E4orf6, p53 and Mdm2. Oncogene 19, 850-857.
Meissner, T., Krause, E., Vinkemeier, U., 2004. Ratjadone and leptomycin B block CRM1-dependent nuclear export by identical mechanisms. FEBS Lett 576, 27-30.
Montefiori, D.C., 2009. Measuring HIV neutralization in a luciferase reporter gene assay. Methods in molecular biology (Clifton, N.J 485, 395-405.
Mutka, S.C., Yang, W.Q., Dong, S.D., Ward, S.L., Craig, D.A., Timmermans, P.B., Murli, S., 2009. Identification of nuclear export inhibitors with potent anticancer in vivo. Cancer research 69, 510-517.
Newlands, E.S., Rustin, G.J., Brampton, M.H., 1996. Phase I trial of elactocin. British journal of cancer 74, 648-649.
Pavlakis, G.N., Stauber, R.H., 1998. Regulatory Proteins of HIV-1. In: Saksena, N. (Ed.), Human Immunodeficiency Viruses: Biology, Immunology and Molecular Biology. Medical SystemsSpA, Genova, pp. 103-122.
Poeta, M.L., Manoal, J., Goldwasser, M.A., Forastiere, A., Benoit, N., Califano, J.A., Ridge, J.A., Goodwin, J., Kenady, D., Saunders, J., Westra, W., Sidransky, D., Koch, M.W., 2007. TP53 mutations and survival in squamous-cell carcinoma of the head and neck. N Engl J. Med 357, 2552-2561.
Stauber, R., Gaitanaris, G.A., Pavlakis, G.N., 1995. Analysis of trafficking of Rev and transdominant Rev proteins in living cells using Green Fluorescent Protein fusions: Transdominant Rev blocks the export of Rev from the nucleus to the cytoplasm. Virology 213, 439-449.
Stauber, R.H., Rabenhorst, U., Rekik, A., Engels, K., Bier, C. and Knauer, S.K., 2006. Nucleocytoplasmic shuttling and the biological activity of mouse survivin are regulated by an active nuclear export signal. Traffic 7(11): 1461-72.
Stauber, R.H., Mann, W., Knauer, S.K., 2007. Nuclear and cytoplasmic Survivin: molecular mechanism, prognostic, and therapeutic potential. Cancer research 67, 5999-6002.
Van Neck, T., Pannecouque, C., Vanstreels, E., Stevens, M., Dehaen, W., Daelemans, D., 2008. Inhibition of the CRM1-mediated nucleocytoplasmic transport by N-azolylacrylates: structure-activity relationship and mechanism of action. Bioorg Med Chem 16, 9487-9497.
Zaidi, S.K., Young, D.W., Javed, A., Pratap, J., Montecino, M., van Wijnen, A., Lian, J.B., Stein, J.L., Stein, G.S., 2007. Nuclear microenvironments in biological control and cancer. Nat Rev Cancer 7, 454-463.

## Claims

1. A compound of formula I, or a pharmaceutically acceptable salt, solvate, hydrate or N-oxide thereof, for use in the treatment or prophylaxis of cancer or viral diseases 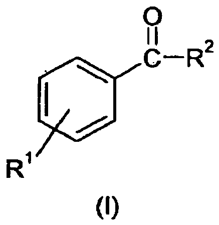 wherein
R¹ is halogen or halo-C₁-C₆-alkyl;
R² is a group of formula II or III; 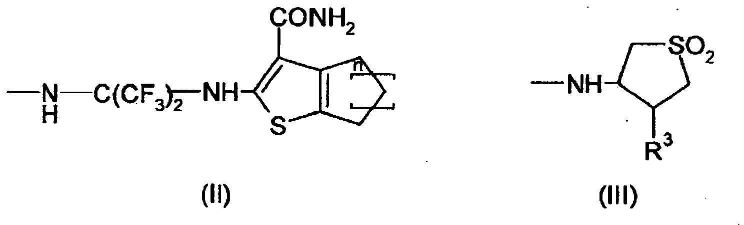 and
R³ is halogen, and
n is 1 or 2.

2. The compound, or pharmaceutically acceptable salt, solvate, hydrate or N-oxide thereof, for use according to claim 1, wherein the compound of formula I has one of formulae la or Ib 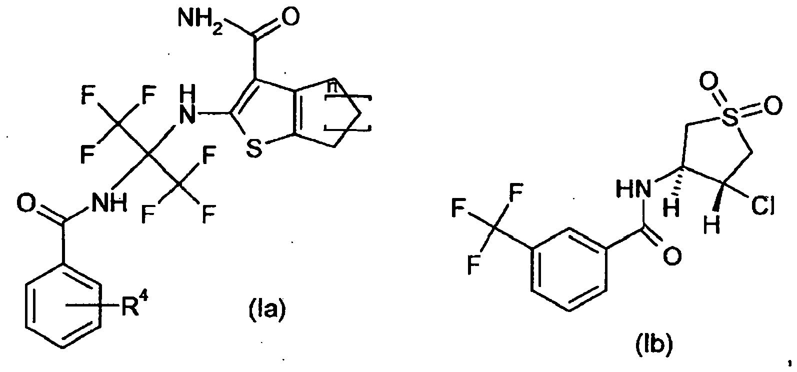 wherein R⁴ is fluorine or chlorine,
and n is 1 or 2.

3. The compound, or pharmaceutically acceptable salt, solvate, hydrate or N-oxide thereof, for use according to claim 1 or 2, wherein the cancer is selected from the group consisting of fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovialoma, mesothelioma, epidermoid carcinoma, Ewing tumor, leiosarcoma, rhabdomyosarcoma, colon carcinoma including colon adenocarcinoma, pancreas cancer, head and neck cancer, mamma carcinoma, ovarial cancer, prostate carcinoma, squamous cell carcinoma, basal cell carcinoma, adenocarcinoma, syringoma, sebaceous carcinoma, papillary carcinoma, papillary adenocarcinoma, cystadenocarcinoma, bone marrow carcinoma, bronchogenic carcinoma, renal cell carcinoma, hepatoma, cholangioma, chorionepithelioma, seminoma, embryo carcinoma, Wilms' tumor, cervix carcinoma, HPV-assodated cervix carcinoma, testicular cancer, lung carcinoma, small-cell lung carcinoma, bladder carcinoma, epithelial carcinoma, glioma, astrocytoma, medulloblastoma, craniopharyngioma, ependymoma, pinealoma, haemangioblastoma, acustic neuroma, oligodendroglioma, meningioma, melanoma, neuroblastoma, retinoblastoma, leukemia such as chronic myeloid leukemia (CML), acute lymphoblastic leukemia (ALL) such as B cell precursor ALL, acute myeloid leukemia (AML), chronic lymphoid leukemia (CLL) and other lymphomas, multiple myeloma, Waldenstroem's macroglobulinemia and heavy-chain disease.

4. The compound, or pharmaceutically acceptable salt, solvate, hydrate or N-oxide thereof, for use according to claim 1 or 2, wherein the pathogen of the viral diseases is selected from the group consisting of adenoviridae, picomaviridae, herpesviridae, hepadnaviridae, flaviviridae, retroviridae, orthomyxoviridae, paramyxoviridae, papillomaviridae, reoviridae, rhabdoviridae, togaviridae, polyomaviridae, bunyaviridae and parvoviridae.

5. The compound, or pharmaceutically acceptable salt, solvate, hydrate or N-oxide thereof, for use according to any one of claims 1 to 4, wherein the compound of formula I, or pharmaceutically acceptable salt, solvate, hydrate or N-oxide thereof, is used in combination with at least one additional anti-cancer agent.

6. The compound, or pharmaceutically acceptable salt, solvate, hydrate or N-oxide thereof, for use according to claim 5, wherein the additional anti-cancer agent is selected from topoisomerase I inhibitors; topoisomerase II inhibitors; alkylating agents; platinum analogues; antimetabolites such as nucleoside mimetics, taxanes; vinca alkaloids; tyrosine kinase inhibitors; proteasome inhibitors; NF-κB-inhibitors; and antibodies, which bind to proteins expressed by cancer cells and inhibit cell replication.

7. The compound, or pharmaceutically acceptable salt, solvate, hydrate or N-oxide thereof, for use according to any one of claims 1 to 4, wherein the compound of formula I, or pharmaceutically acceptable salt, solvate, hydrate or N-oxide thereof, is used in combination with at least one additional antiviral agent.

8. Pharmaceutical composition comprising at least one compound of formula I as defined in claim 1 or 2 or a pharmaceutically acceptable salt, solvate, hydrate or N-oxide thereof.

9. An *in vitro* method for inhibiting the CRM1 dependent export of proteins from the cell nucleus by contacting the cell with a compound of formula I as defined in claim 1 or 2 or a pharmaceutically acceptable salt, solvate, hydrate or N-oxide thereof.

## Patentansprüche

1. Verbindung der Formel I, oder ein pharmazeutisch annehmbares Salz, Solvat, Hydrat oder N-Oxid davon, zur Verwendung bei der Behandlung oder Prophylaxe von Krebs oder Viruserkrankungen 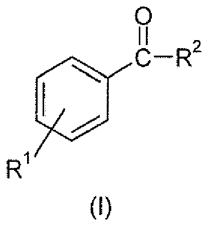 worin
R¹ für Halogen oder Halo-C₁-C₆-alkyl steht;
R² eine Gruppe der Formel II oder III ist; 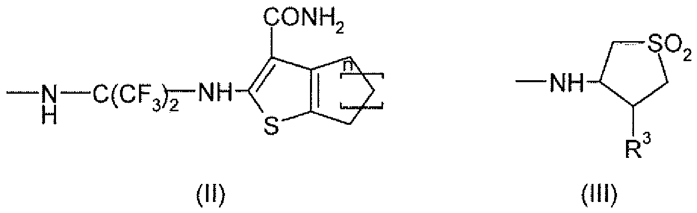 und
R³ für Halogen steht, und
n für 1 oder 2 steht.

2. Die Verbindung, oder das pharmazeutisch annehmbare Salz, Solvat, Hydrat oder N-Oxid davon, zur Verwendung gemäß Anspruch 1, wobei die Verbindung der Formel I einer der Formeln Ia or Ib entspricht 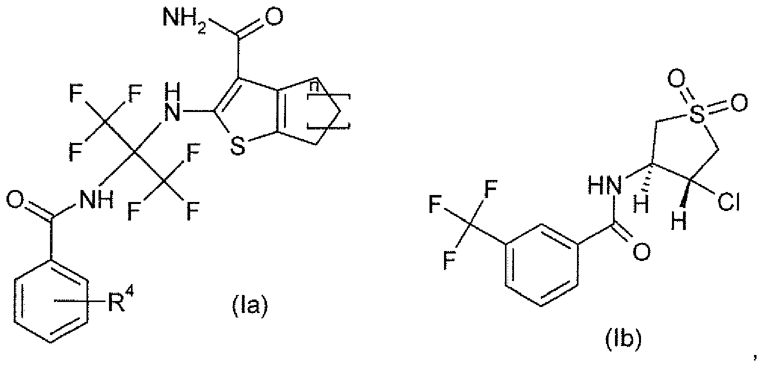 worin
R⁴ für Fluor oder Chlor steht, und
n für 1 oder 2 steht.

3. Die Verbindung, oder das pharmazeutisch annehmbare Salz, Solvat, Hydrat oder N-Oxid davon, zur Verwendung gemäß Anspruch 1 oder 2, wobei der Krebs ausgewählt ist unter Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviatom, Mesotheliom, epidermoidem Karzinom, Ewing-Tumor, Leiosarkom, Rhabdomyosarkom, Darmkarzinom einschließlich Darm-Adenokarzinom, Pankreaskrebs, Kopf- und Halskrebs, Mammakarzinom, Eierstockkrebs, Prostatakarzinom, Plattenepithelkarzinom, Basatzellkarzinom, Adenokarzinom, Syringom, Talgdrüsenkarzinom, papillärem Karzinom, papillärem Adenokarzinom, Zystadenokarzinom, Knochenmarkkarzinom, Bronchialkarzinom, Nierenzellkarzinom, Hepatom, Cholangiom, Chorionepitheliom, Seminom, Embryokarzinom, Wüms-Tumor, Zervixkarzinom, HPV-assoziiertem Zervixkarzinom, Hodenkrebs, Lungenkarzinom, kleinzelligem Lungenkarzinom, Harnblasenkarzinom, Epithelkarzinom, Gliom, Astrocytom, Medulloblastom, Kraniopharyngiom, Ependymom, Pinealom, Haemangioblastom, Akustikusneurinom, Oligodendrogliom, Meningiom, Melanom, Neuroblastom, Retinoblastom, Leukämie wie zum Beispiel chronischer myeloischer Leukämie (CML), akuter lymphoblastischer Leukämie (ALL) wie zum Beispiel B-Zell-Vorläufer-ALL, akuter myeloischer Leukämie (AML), chronischer lymphoider Leukämie (CLL) und anderen Lymphomen, multiplem Myelom, Waldenströms Makroglobulinämie und Schwerkettenkrankheit.

4. Die Verbindung, oder das pharmazeutisch annehmbare Salz, Solvat, Hydrat oder N-Oxid davon, zur Verwendung gemäß Anspruch 1 oder 2, wobei das Pathogen der Viruserkrankungen ausgewählt ist unter Adenoviren, Picornaviren, Herpesviren, Hepadnaviren, Flaviviren, Retroviren, Orthomyxoviren, Paramyxoviren, Papillomaviren, Reoviren, Rhabdoviren, Togaviren, Polyomaviren, Bunyaviren und Parvoviren,

5. Die Verbindung, oder das pharmazeutisch annehmbare Satz, Solvat, Hydrat oder N-Oxid davon, zur Verwendung gemäß einem der Ansprüche 1 bis 4, wobei die Verbindung der Formel I, oder das pharmazeutisch annehmbare Salz, Solvat, Hydrat oder N-Oxid davon, in Kombination mit mindestens einem weiteren Antikrebs-Wirkstoff verwendet wird.

6. Die Verbindung, oder das pharmazeutisch annehmbare Salz, Solvat, Hydrat oder N-Oxid davon, zur Verwendung gemäß Anspruch 5, wobei der weitere Antikrebs-Wirkstoff ausgewählt ist unter Topoisomerase-I-Inhibitoren; Topoisomerase-IIInhibitoren; Alkylierungsmitteln; Platinanaloga; Antimetaboliten wie zum Beispiel Nukleosidmimetika, Taxanen; Vinca-Alkaloiden; Tyrosinkinase-Inhibitoren; Proteasom-Inhibitoren; NF-κB-Inhibitoren; und Antikörpern, die an von Krebszellen exprimierte Proteine binden und die Zellreplikation inhibieren.

7. Die Verbindung, oder das pharmazeutisch annehmbare Salz, Solvat, Hydrat oder N-Oxid davon, zur Verwendung gemäß einem der Ansprüche 1 bis 4, wobei die Verbindung der Formel I, oder das pharmazeutisch annehmbare Salz, Solvat, Hydrat oder N-Oxid davon, in Kombination mit mindestens einem weiteren antiviralen Wirkstoff verwendet wird.

8. Pharmazeutische Zusammensetzung, die mindestens eine Verbindung der Formel I, die wie in Anspruch 1 oder 2 definiert ist, oder ein pharmazeutisch annehmbares Salz, Solvat, Hydrat oder N-Oxid davon, umfasst.

9. *In-vitro*-Verfahren zur inhibition des CRM1-abhängigen Exports von Proteinen aus dem Zellkern, wobei die Zelle mit einer Verbindung der Formel I, die wie in Anspruch 1 oder 2 definiert ist, oder einem pharmazeutisch annehmbaren Salz, Solvat, Hydrat oder N-Oxid davon, in Kontakt gebracht wird.

## Revendications

1. Composé de la formule I, ou sel, solvate, hydrate ou N-oxyde de celui-ci pharmaceutiquement acceptable pour une utilisation dans le traitement ou la prophylaxie du cancer ou de maladies virales. 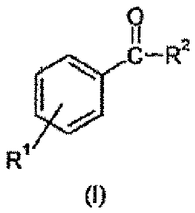 où
R¹ est un atome d'halogène ou un groupe halo-(alkyle en C₁-C₆) ;
R² est un groupe de la formule II ou III ; 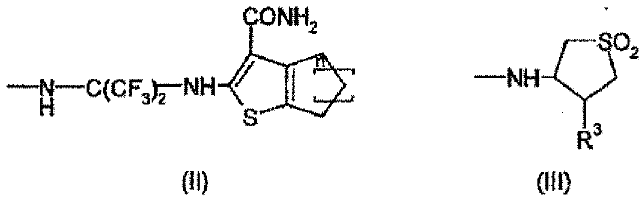 et
R³ est un atome d'halogène, et
n est égal à 1 ou 2.

2. Composé, ou sel, solvate, hydrate ou N-oxyde de celui-ci pharmaceutiquement acceptable pour une utilisation selon la revendication 1, dans lequel le composé de la formule 1 présente une des formules Ia ou Ib. 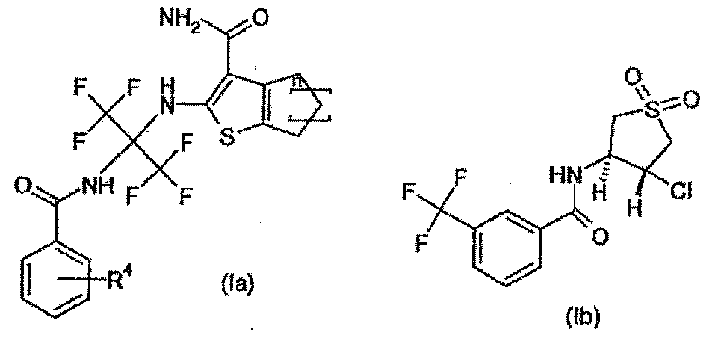
où R⁴ est le fluore ou le chlore,
et n est égal à 1 ou 2.

3. Composé, ou sel, solvate, hydrate ou N-oxyde de celui-ci pharmaceutiquement acceptable pour une utilisation selon la revendication 1 ou 2, dans lequel le cancer est choisi dans le groupe constitué d'un fibrosarcome, d'un myxosarcome, d'un liposarcome, d'un chondrosarcome, d'un sarcome ostéogénique, d'un chordome, d'un angiosarcome, d'un endothéliosarcome, d'un lymphangiosarcome, d'un lymphangloendothéliosarcome, d'un synovialome, d'un mésothéliome, d'un carcinome épidermique, d'une tumeur d'Ewing, d'un leiomyosarcome, d'un rhabdomyosarcome, d'un cancer du colon comprenant un adénosarcome du colon, d'un cancer du pancréas, d'un cancer de la tête et du cou, d'un cancer du sein, d'un cancer des ovaires, d'un cancer de la prostate, d'un carcinome cellulaire squameux, d'un carcinome basocellulaire, d'un adénosarcome, d'un syringome, d'un carcinome sébacé, d'un adénocarcinome papillaire, d'un cystadénocarcinome, d'un cancer de la moelle osseuse, d'un cancer bronchopulmonaire, d'un cancer des cellules rénales, d'un hépatome, d'un cholangiome, d'un chorioépithéliome, d'un séminome, d'un carcinome embryonnaire, d'une tumeur de Wilms, d'un cancer du col de l'utérus , d'un cancer du col de l'utérus associé à HPV, d'un cancer des testicules, d'un cancer du poumon, d'un cancer pulmonaire à petites cellules, d'un cancer de la vessie, d'un épithéliome, d'un gliome, d'un astrocytome, d'un médulloblastome, d'un craniopharyngiome, d'un épendymome, d'un pinéalome, d'un hémangioblastome, d'un neurinome acoustique, d'un oligodendrogliome, d'un méningiome, d'un mélanome, d'un neuroblastome, d'un rétinoblastome, d'une leucémie, telle qu'une leucémie myéloïde chronique (CML), une leucémie lymphoblastique aiguë (ALL), telle qu'une ALL à précurseur de cellule B, une leucémie myéloïde aiguë (AML), une leucémie lymphoïde chronique (CLL) et d'autres lymphomes, d'un myélome multiple, d'une macroglobulinémie de Waldenstrom et d'une maladie de Franklin.

4. Composé, ou sel, solvate, hydrate ou N-oxyde de celui-ci pharmaceutiquement acceptable pour une utilisation selon la revendication 1 ou 2, dans lequel le pathogène des maladies virales est choisi dans le groupe constitué d'adénovirus, de picornavirus, de virus de l'herpès, de virus hépatique, de flavivirus, de rétrovirus, d'orthomyxovirus, de paramyxovirus, de papillomavirus, de réovirus, de rhabdovirus, de togavirus, de polyomavirus, de bunyavirus et de parvovirus.

5. Composé, ou sel, solvate, hydrate ou N-oxyde de celui-ci pharmaceutiquement acceptable pour une utilisation selon l'une quelconque des revendications 1 à 4, dans lequel le composé de la formule I, ou le sel, solvate, hydrate ou N-oxyde de celui-ci pharmaceutiquement acceptable est utilisé en combinaison avec au moins un agent anti-cancer supplémentaire.

6. Composé, ou sel, solvate, hydrate ou N-oxyde de celui-ci pharmaceutiquement acceptable pour une utilisation selon la revendication 5, dans lequel l'agent anticancer supplémentaire est choisi parmi des inhibiteurs de topoisomérase I ; des inhibiteurs de topoisomérase II, des agents d'alkylation ; des analogues du platine ; des antimétabolites, tels que des mimétiques nucléosidiques, des taxanes ; des alcaloïdes vinca ; des inhibiteurs de tyrosine kinase ; des inhibiteurs de protéasome ; des inhibiteurs de NF-κB ; et des anticorps, lesquels se lient à des protéines exprimées par des cellules cancéreuses et inhibent la réplication des cellules.

7. Composé, ou sel, solvate, hydrate ou N-oxyde de celui-ci pharmaceutiquement acceptable pour une utilisation selon l'une quelconque des revendications 1 à 4, dans lequel le composé de la formule I, ou le sel, solvate, hydrate ou N-oxyde de celui-ci pharmaceutiquement acceptable est utilisé en combinaison avec au moins un agent antiviral supplémentaire.

8. Composition pharmaceutique comprenant au moins un composé de la formule 1 selon la revendication 1 ou 2 ou un sel, solvate, hydrate ou N-oxyde de celui-ci pharmaceutiquement acceptable.

9. Procédé *in vitro* pour inhiber l'export dépendant de CRM1 de protéines à partir du noyau cellulaire par mise en contact de la cellule avec un composé de la formule I selon la revendication 1 ou 2 ou un sel, solvate, hydrate ou N-oxyde de celui-ci pharmaceutiquement acceptable.
